(19) Europäisches Patentamt
European Patent Office
Office européen des brevets

(11) EP 1 269 975 B1

(12) **EUROPÄISCHE PATENTSCHRIFT**

(45) Veröffentlichungstag und Bekanntmachung des Hinweises auf die Patenterteilung:
**22.10.2003 Patentblatt 2003/43**

(51) Int Cl.$^7$: **A61K 7/13**

(21) Anmeldenummer: **02014195.8**

(22) Anmeldetag: **26.06.2002**

(54) **Haarfärbemittel**

Hair colourant

Teinture pour cheveux

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **29.06.2001 DE 10131539**

(43) Veröffentlichungstag der Anmeldung:
**02.01.2003 Patentblatt 2003/01**

(73) Patentinhaber: **KPSS-Kao Professional Salon Services GmbH**
**64297 Darmstadt (DE)**

(72) Erfinder:
- **Nöcker, Bernd, Dr.**
  **64372 Ober-Ramstadt (DE)**
- **Wilz, Rüdiger**
  **64319 Pfungstadt (DE)**
- **Ghiasi, Fariba**
  **60316 Frankfurt (DE)**
- **Garbe, Barbara**
  **64625 Bensheim (DE)**

(56) Entgegenhaltungen:
**EP-A- 1 059 077**
**DE-A- 19 926 167**
**US-A- 5 626 868**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 269 975 B1

## Beschreibung

Die Erfindung betrifft ein Haarfärbemittel auf wäßriger Basis, das mindestens einen direktziehenden Haarfarbstoff enthält.
Derartige Haarfärbemittel sind seit langem bekannt.
Sie enthalten meistens mehrere anionische oder kationische direktziehende Farbstoffe und bedürfen, im Gegensatz zu den permanenten Haarfärbemitteln auf Basis von Oxidationsfarbstoffvorprodukten, keiner vorherigen Entwicklung mit Oxidationsmitteln.

Diese direktfärbenden Zusammensetzungen werden entweder zusammen mit oberflächenaktiven Mitteln als sogenannte Tönungsshampoos appliziert, oder als Lotionen, Emulsionen oder verdickte Lösungen, d.h., Gele, auf das Haar aufgebracht.

Die Erfindung geht daher von der Aufgabenstellung aus, ein Haarfärbemittel auf wäßriger Basis zu schaffen, das als Lotion, Emulsion, Lösung, Gel, Suspension bzw. auch, unter Zusatz von Treibmitteln, als Aerosolzusammensetzung auf das Haar aufgebracht werden kann, das optimale haarkonditionierende Eigenschaften aufweist, und sowohl mit anionischen als auch kationischen direktziehenden Haarfarbstoffen ausgezeichnete färberische Eigenschaften zeigt.

Diese direktziehenden Haarfärbemittel sind seit Jahrzehnten bekannt und bewährt, gleichwohl jedoch auch noch verbesserungsfähig, insbesondere hinsichtlich der Eigenschaften der durch sie erzielten Haarfärbungen, vor allem Farbintensität und Farbstabilität, d.h. Beständigkeit gegen Haarwäsche, Dauerwellbehandlung und Umwelteinflüsse wie UV-Strahlen und chemische Beeinflussungen, insbesondere bei geschädigtem Haar.

Gelöst wird diese Aufgabe durch den Zusatz von 0,001 bis 10 Gew.-%, berechnet auf die Gesamtzusammensetzung, mindestens eines Ubichinons der Formel

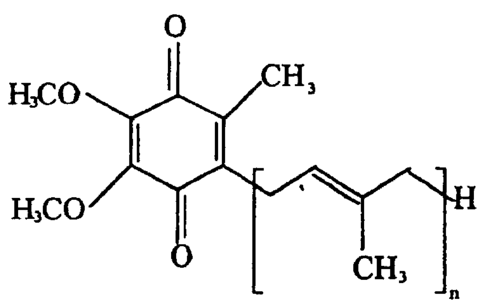

(I)

,

worin n eine Zahl von 1 bis 10 bedeutet.
Bevorzugte Ubichinone sind solche mit n = 6 bis 10, insbesondere Ubichinon 50, worin n 10 bedeutet, auch unter der Bezeichnung "Coenzym Q 10" bekannt.
Die Verwendung von "Coenzym Q 10" in kosmetischen Mitteln, insbesondere in Haarpflegemitteln, ist an sich bekannt.
Die EP 0 751 762 B beschreibt Haarpflegemittel, die ein oder mehrere Ubichinone, insbesondere Coenzym Q 6 und Coenzym Q 10, in Kombination mit Retinolen, Retinalen und β-Carotin, zur Behandlung der Hautalterung enthalten.
Aus der DE 199 26 167 A1 sind Stylingmittel auf Wasserbasis bekannt, die ein oder mehrere Biochinone wie Ubichinone und Plastochinone zum Schutz der Kopfhaut und des Haares vor unerwünschten Oxidationsprozessen enthalten.
Die DE 199 26 168 A1 offenbart Stylingmittel auf alkoholischer Basis mit Biochinonen, insbesondere Coenzym Q 10.
Die DE 199 26 170 A1 betrifft die Verwendung eines oder mehrerer Biochinone wie Ubichinone in kosmetischen Haarreinigungsmitteln.
Schließlich beschreibt die DE 199 26 156 A1 die Verwendung von Biochinonen wie Ubichinon zur Herstellung von kosmetischen Zubereitungen zur Verbesserung der Haarstruktur, insbesondere der Kämmbarkeit des Haares, also haarkonditionierenden Zusammensetzungen.

Gegenüber diesem Stand der Technik war die farbverbessernde Wirkung des Zusatzes von Ubichinonen in Haarfärbemitteln mit direktziehenden Farbstoffen, insbesondere bei Anwendung auf vorgeschädigtem Haar, wobei zusätzlich auch noch dessen kosmetische Eigenschaften wie Glanz, Griff und Sprungkraft verbessert werden, nicht vorhersehbar.

Die bevorzugte Einsatzkonzentration der Coenzyme Q1 bis Q 10 beträgt etwa 0,005 bis 5, insbesondere etwa 0,01 bis 2,5, besonders bevorzugt etwa 0,05 bis 1 Gew.-%.

Der Anteil der direktziehenden Farbstoffe in den erfindungsgemäßen Zusammensetzungen ist variabel und liegt zwischen etwa 0,005 bis etwa 5, vorzugsweise 0,01 bis 2,5, insbesondere 0,1 bis 1 Gew.-% des Mittels.

Als direktziehende Haarfarbstoffe können im Prinzip alle für diesen Zweck vorgeschlagenen kationischen

Farbstoffe verwendet werden.

Bevorzugt sind die sogenannten "Arianor"-Farbstoffe; vgl. K. Schrader, "Grundlagen und Rezepturen der Kosmetika", 2. Aufl. (1989), S. 811.

Besonders geeignete basische (kationische) Farbstoffe sind:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 12 | C.I.-No. 48,070; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |

Selbstverständlich ist auch die Verwendung entsprechender direktziehender Pflanzenfarbstoffe oder auch anionischer (saurer) direktziehender Haarfarbstoffe möglich.

Diese werden üblicherweise ebenfalls in einer Menge von etwa 0,005 bis etwa 5, vorzugsweise etwa 0,05 bis etwa 2,5, insbesondere etwa 0,1 bis etwa 1 Gew.-%, berechnet auf die Gesamtzusammensetzung des Mittels, das als Lösung, Dispersion, Emulsion, Gel oder Aerosolpräparat zur direkten Anwendung vorliegt, eingesetzt.

Als geeignete anionische Farbstoffe können beispielsweise Verwendung finden:

| | |
|---|---|
| Acid Black 1, | C.I.-No. 20,470; |
| Acid Blue 1, | C.I.-No. 42,045; |
| Food Blue 5, | C.I.-No. 42,051; |
| Acid Blue 9, | C.I.-No. 42,090; |
| Acid Blue 74, | C.I.-No. 73,015; |
| Acid Red 18, | C.I.-No. 16,255; |
| Acid Red 27, | C.I.-No. 16,185; |
| Acid Red 87, | C.I.-No. 45,380; |
| Acid Red 92, | C.I.-No. 45,410; |
| Acid Orange 7, | C.I.-No. 15,510; |
| Acid Violet 43, | C.I.-No. 60,730; |
| Acid Yellow 1, | C.I.-No. 10,316; |
| Acid Yellow 23, | C.I.-No. 19,140; |
| Acid Yellow 3, | C.I.-No. 47,005; |
| Food Yellow No. 8, | C.I.-No. 14,270; |
| D&C Brown No. 1, | C.I.-No. 20,170 |
| D&C Green No. 5, | C.I.-No. 61,570; |
| D&C Orange No. 4, | C.I.-No. 15,510; |
| D&C Orange No. 10, | C.i.-No 45,425:1; |
| D&C Orange No. 11, | C.I.-No. 45,425; |

(fortgesetzt)

| | |
|---|---|
| D&C Red No. 21, | C.I.-No. 45,380:2; |
| D&C Red No. 27, | C.I.-No. 45,410:1; |
| D&C Red No. 33, | C.I.-No. 17,200; |
| D&C Yellow No. 7, | C.I.-No. 45,350:1; |
| D&C Yellow No. 8, | C.I.-No. 45,350; |
| FD&C Red No. 4, | C.I.-No. 14,700; |
| FD&C Yellow No. 6, | C.I.-No. 15,985. |

Auch pflanzliche Farbstoffe können allein oder in Kombination mit synthetischen Direktziehern Verwendung finden, beispielsweise Henna (rot oder schwarz), Alkannawurzel, Laccainsäure (Stocklack), Indigo, Blauholzpulver, Krappwurzel- und Rhabarberwurzelpulver, etc.

Das erfindungsgemäße Haarfärbemittel, dessen pH-Wert bei etwa 10 bis 1 liegt, kann auch oberflächenaktive Substanzen enthalten.
Solche können anionisch, nichtionisch, kationisch oder amphoter bzw. zwitterionisch sein.

Geeignete nichtionische Tenside sind Verbindungen aus der Klasse der Alkylpolyglucoside mit der allgemeinen Formel

$$R\text{-}O\text{-}(CH_2CH_2O)_n\text{-}Z_x,$$

worin R eine Alkylgruppe mit 8 bis 20, vorzugsweise 10 bis 14 Kohlenstoffatomen, $Z_x$ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.

Weitere geeignete nichtionische Tenside in den erfindungsgemäßen Mitteln sind $C_{10}$-$C_{22}$-Fettalkoholethoxylate.

Besonders geeignete $C_{10}$-$C_{22}$-Fettalkoholether sind die unter den Trivialnamen "Laureth", "Myristeth", "Oleth", Ceteth", "Deceth", "Steareth" und "Ceteareth" nach der CTFA-Nomenklatur mit Anfügung der Zahl der Ethylenoxid-Moleküle bezeichneten Alkylpolyglykolether, z.B. "Laureth-16":

Der durchschnittliche Ethoxylierungsgrad liegt dabei zwischen etwa 2,5 und etwa 25, vorzugsweise etwa 10 und etwa 20.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics®" im Verkehr sind.

Weitere zusätzlich einsetzbare Tenside sind Aminoxide.
Solche Aminoxide gehören seit langem zum Stand der Technik, beispielsweise $C_{12}$-$C_{18}$-Alkyldimethylaminoxide wie Lauryldimethylaminoxid, $C_{12}$-$C_{18}$-Alkylamidopropyloder ethylaminoxide, $C_{12}$-$C_{18}$-Alkyldi(hydroxyethyl)- oder -(hydroxypropyl)aminoxide, oder auch Aminoxide mit Ethylenoxid- undloder Propylenoxidgruppen in der Alkylkette.

Geeignete Aminoxide sind beispielsweise unter den Bezeichnungen "Ammonyx®", "Aromox®", oder "Genaminox®" im Handel.

Weitere fakultative Tensidbestandteile sind Fettsäuremono- und dialkanolamide, wie Cocosfettsäuremonoethanolamid und Myristinfettsäuremonoisopropanolamid.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natrium-cocoamphopropionat und -acetat haben sich als geeignet erwiesen.

In einzelnen können Betaine der Struktur

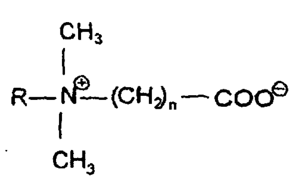 und 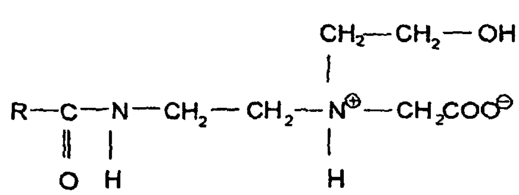 ,

wobei R eine $C_8$-$C_{18}$-Alkylgruppe und n 1 bis 3 bedeuten,

Sulfobetaine der Struktur wobei R eine $C_8$-$C_{18}$-

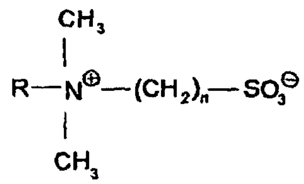

Alkylgruppe und n 1 bis 3 bedeuten,

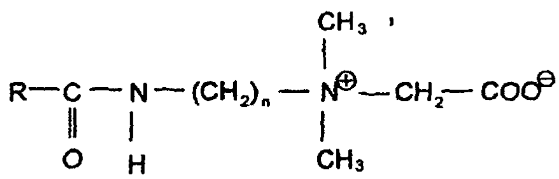

,

wobei R eine $C_8$-$C_{18}$-Alkylgruppe und n 1 bis 3 bedeuten, verwendet werden.

Bevorzugt sind Fettsäureamidoalkylbetaine, insbesondere Cocoamidopropylbetain, und Cocoamphoacetat und-propionat, insbesondere deren Natriumsalze.

Besonders bevorzugt sind Gemische aus Cocoamidopropylbetain und Cocoamphoacetat, insbesondere im Gewichtsverhältnis 3:1 bis 1:3, vor allem 2:1 bis 1:1.

Geeignete kationische Tenside sind beispielsweise langkettige quaternäre Ammoniumverbindungen, die allein oder im Gemisch miteinander eingesetzt werden können, wie Cetyltrimethylammoniumchlorid, Dimethyldiethylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxy-ethyl)alkylammoniumphosphat, Cetylpyridiniumchlorid, etc. Gut geeignet sind auch die in der EP-A 472 107 geoffenbarten quaternären Ammoniumsalze.

Weitere geeignete langkettige Ammoniumverbindungen sind Esterquats der allgemeinen Formel (I)

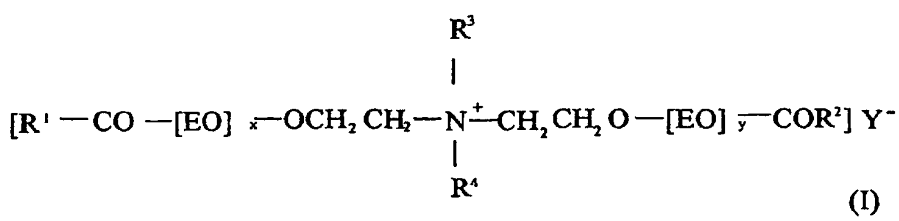

(I)

in der $R^1$ und $R^2$ für eine gegebenenfalls hydroxysubstituierte $C_8$-$C_{22}$-Alkyloder Alkenylgruppe, $R^3$ und $R^4$ für eine $C_1$-$C_3$-Alkylgruppe oder eine Gruppe $-CH_2-CH_2-O-[EO]_zH$ sowie x,y und z für 0 bis 5 und $Y^-$ für ein Anion stehen.

Eine besonders bevorzugte Verbindung der Formel I ist im Rahmen der Erfindung eine solche, in der die Reste $R^1$ und $R^2$ jeweils eine Oleylgruppe oder eine $C_{12}$-$C_{18}$-Alkylgruppe, der Rest $R^3$ eine Methylgruppe und der Rest $R^4$ eine Gruppe $-CH_2-CH_2-O-[EO]_z-H$ bedeuten.

Das Anion $Y^-$ ist vorzugsweise ein Halogenid wie $Cl^-$ oder $Br^-$, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat$^R$", "Dehyquart$^R$F30" und "Tetranyl$^R$" im Handel.

Der Einsatz dieser "Esterquats" in Haarpflegemitteln ist an sich bekannt und beispielsweise in der WO-A 93/10748, der WO-A 92/06899 und der WO-A 94/16677 beschrieben.

Geeignet sind auch Amidoquats der allgemeinen Formel (II)

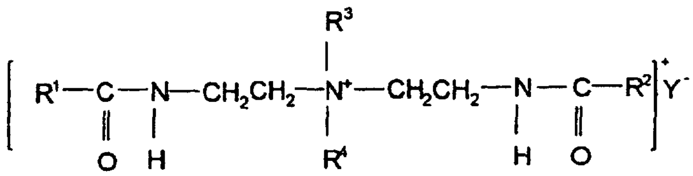

(II),

in der $R^1$ und $R^2$ jeweils für eine gegebenenfalls hydroxysubstituierte $C_8$-$C_{22}$-Alkyloder Alkenylgruppe, $R^3$ und $R^4$ für eine $C_1$-$C_3$-Alkylgruppe oder eine Gruppe-$CH_2$-$CH_2$-O-$[EO]_x$-H, sowie x für 0 bis 5, Y für ein Anion stehen.

Es können selbstverständlich auch Gemische aus den verschiedenen Tensiden, soweit sie miteinander kompatibel sind, verwendet werden.

Ein weiterer wünschenswerter Bestandteil der erfindungsgemäßen Färbemittel-Zusammensetzungen ist ein $C_3$-$C_6$-Alkandiol bzw. dessen Ether, insbesondere ein Mono-$C_1$-$C_3$-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Färbemittel-Zusammensetzung.

Neben den $C_3$-$C_6$-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Färbemittels.

Diese Mittel können auch weitere Pflegemittel wie Öle und Fette enthalten. Solche sind beispielsweise Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.
Falls es sich bei den erfindungsgemäßen Mitteln um Emulsionen handelt, enthalten diese selbstverständlich die üblichen Emulgatoren.

Der pH-Wert dieser erfindungsgemäßen Mittel liegt vorzugsweise im Bereich zwischen etwa 2 und 9, beispielsweise 2,5 und 7, insbesondere etwa 3 und 5,0.

Falls es sich um Tönungsshampoos handelt, können diese natürlich, neben den weiter oben aufgezählten oberflächenaktiven Substanzen, auch anionische Tenside enthalten.
Geeignete anionaktive Tenside sind in einer Menge von mindestens 5 bis etwa 50 Gew.-%, vorzugsweise 10 bis 25 Gew.-% der Zusammensetzung enthalten.

Dabei handelt es sich um solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten $C_{10}$-$C_{18}$-Alkylsulfate und insbesondere die entsprechenden Ethersulfate, beispielsweise $C_{12}$-$C_{14}$-Alkylethersuifat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.
Weiterhin geeignete anionische Tenside sind $\alpha$-Olefinsulfonate bzw. deren Salze und insbesondere Alkalisalze von Sulfobernsteinsäurehalbestern, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

$$R - (C_2H_4O)_n - O - CH_2\ COOX,$$

worin R eine $C_8$-$C_{20}$-Alkylgruppe, vorzugsweise eine $C_{12}$-$C_{14}$-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel

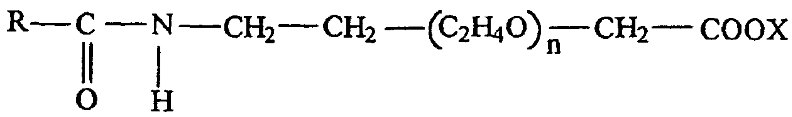

worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO®" und "AKYPO-SOFT®".

Auch $C_8$-$C_{20}$-Acylisethionate können, allein oder im Gemisch mit anderen Tensiden, eingesetzt werden, ebenso Sulfofettsäuren und deren Ester.

Es können auch Mischungen aus mehreren anionischen Tensiden eingesetzt werden, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monographie von K. Schrader, "Grundlagen und Rezepturen der Kosmetika", 2. Aufl. (1989, Hüthig Buchverlag), S. 683 bis 691.

Der bevorzugte Mengenbereich an anionischen Tensiden in Tönungsshampoos gemäß der Erfindung liegt zwischen etwa 5 und etwa 30 Gew.-%, insbesondere bei etwa 7,5 bis etwa 25 Gew.-%, besonders bevorzugt bei etwa 10 bis etwa 20 Gew.-%, berechnet auf die Gesamtzusammensetzung des Mittels, je nachdem, ob es sich um Konzentrate handelt.

Als bevorzugte Ausführungsform enthalten die erfindungsgemäßen Tönungsshampoos vorzugsweise ein Gemisch aus einem der genannten anionischen Tenside und mindestens einer $C_8$-$C_{22}$-Acylaminocarbonsäure bzw. deren wasserlöslichen Salzen, vorzugsweise in einer Menge von 0,5 bis 10, insbesondere 1 bis 7,5 Gew.-%, berechnet auf die Gesamtzusammensetzung.

Besonders bevorzugt ist das N-Lauroylglutamat, insbesondere als Natriumsalz. Weitere geeignete N-Acylaminocarbonsäuren sind beispielsweise N-Lauroylsarcosinat, N-$C_{12}$-$C_{18}$-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze.

Die folgenden Beispiele dienen der Illustration der Erfindung.

| Beispiel 1 | |
|---|---|
| Polyquaternium-6 | 0,60 (Gew.-%) |
| Cetrimoniumchlorid | 0,80 |
| Ethanol | 7,40 |
| Parfum | 0,30 |
| PVP/VA-Copolymer | 0,70 |
| Basic Blue 99 (C.I.-No 56059) | 0,04 |
| Basic Brown 16 (C.I.-No. 12250) | 0,36 |
| HC-Red 3 | 0,17 |
| HC-Yellow 5 | 0,33 |
| Organopolysiloxan/Polyethyloxazolin-Pfropfcopolymerisat (Organopolysiloxan A1 der EP-A 640 643) | 0,50 |
| Coenzym Q10 | 0,05 |
| | |
| Wasser | ad 100,00 |
| pH-Wert: | 6,5 |

Bei der Haarfärbung wurde ein glänzender, gleichmäßiger Kupferrotton erhalten, der nach fünf Haarwäschen noch deutlich war.

Das Weglassen des Coenyzm Q10 führte nicht nur zu einer weniger intensiven Haarfärbung, sondern auch zu verschlechterten Haareigenschaften, wie rauherer Griff, reduzierte Naß- und Trockenkämmbarkeit sowie geringere Spannkraft.

| **Beispiel 2** | |
|---|---|
| Vinylpyrrolidon/Vinylacetat-Copolymer (Luviskol[R]VA64W) | 1,60 (Gew.-%) |
| Xanthan-Gum (Keltrol[R]) | 1,50 |
| Parfum | 0,30 |
| Phenoxyethanol | 0,10 |
| 1,2-Propandiol | 0,50 |
| Ethanol | 0,75 |
| Natriumhydroxid, 32%-ig | 0,20 |
| Basic Blue 99 (C.I.-No 56 059) | 0,10 |
| Basic Brown 16 (C.I.-No. 12 250) | 0,18 |
| Basic Brown 17 (C.I.-No. 12 251) | 0,32 |
| Basic Yellow 57 (C.I.-No. 12 719) | 0,36 |
| Coenzym Q10 | 0,05 |
| Coenzym Q6 | 0,05 |
| Wasser | ad 100,00 |
| pH-Wert: | 8,6 |

Bei der Haarfärbung entsprechend dem in Beispiel 1 beschriebenen Verfahren wurde eine glänzende, über mehrere Haarwäschen stabile, gleichmäßige Goldfärbung erzielt.
Das Haar wies ausgezeichnete Konditioniereigenschaften auf.

Weglassen der Coenzyme Q10 und Q6 resultierte nicht nur in verschlechterten Haareigenschaften wie rauherer Griff, reduzierter Naß- und Trockenkämmbarkeit sowie geringerer Spannkraft, sondern vor allem auch in deutlich reduzierter Farbintensität, Gleichmäßigkeit der Färbung und weniger Glanz.

| **Beispiel 3** | |
|---|---|
| Dimethicone Copolyol | 1,50 (Gew.-%) |
| Ethanol | 5,00 |
| Propylencarbonat | 25,00 |
| Milchsäure, 90%-ig | 5,00 |
| Natriumhydroxid, 32%-ig | 0,20 |
| Quaternäres Dimethylaminoethylmethacrylat-Homopolymerisat (Polyquaternium-37;50%-ig in einem Propylenglykoldicaprat-dicaprylat PPG-1-Trideceth-6 Gemisch; Salcare[R]SC96) | 3,50 |
| Acid Orange 7 (C.I.-No. 15510) | 0,15 |
| Acid Yellow 3 (C.I.-No. 47005) | 0,10 |
| Acid Violet 43 (C.I.-No. 60 730) | 0,25 |
| Organopolysiloxan/Polyethyloxazolin-Pfropfcopolymerisat (Organopolysiloxan A1 der EP-A 640 643) | 0,10 |
| Coenzym Q10 | 0,09 |
| Wasser | ad 100,00 |
| pH-Wert: | 3,0 |

Die Zusammensetzung wurde auf nasses Haar aufgebracht.
Nach etwa zwanzigminütiger Einwirkung wurde gewaschen, gespült und getrocknet. Es wurde ein glänzend und gleichmaßig mittelbraun gefärbtes Haar, das leicht kämmbar war, einen vollen Griff und Spannkraft aufwies, erhalten. Die Färbung blieb über vier Haarwäschen haltbar.

Das Weglassen des Coenzyms führte zu einer deutlich weniger intensiven und gleichmäßigen Färbung mit

verringertem Glanz.
Darüberhinaus waren sowohl die Naß- als auch die Trockenkämmbarkeit sowie der Griff des Haares deutlich schlechter.

| **Beispiel 4** | |
|---|---|
| Menschliches Haar wurde mit einem Tönungsshampoo der Zusammensetzung | |
| $C_{12}$-$C_{14}$-Alkylpolyglucosid (P.D.~1,45) | 10,00 (Gew.-%) |
| Cocoamidopropylbetain | 2,00 |
| PEG-3-distearat | 2,00 |
| Coenzym Q10 | 0,08 |
| Parfum | 0,40 |
| Citronensäure, Konservierungsmittel | q.s. |
| Basic Brown 16 | 0,10 |
| Basic Brown 17 | 0,10 |
| Basic Blue 99 | 0,02 |
| Wasser | ad 100,00 |
| pH-Wert: | 7,0 |

gewaschen, gespült und getrocknet.
Es wurde eine ausdrucksvolle, glänzende, dauerhafte, intensive, gleichmäßige Braunfärbung erzielt, die auch nach drei Haarwäschen noch in gutem Zustand war.
Das Weglassen des Coenzyms führte zu einer deutlich weniger intensiven und gleichmäßigen Färbung mit verringertem Glanz.
Darüberhinaus waren sowohl die Naß- als auch die Trockenkämmbarkeit sowie der Griff des Haares deutlich schlechter.

| | |
|---|---|
| Dimethicone Copolyol | 1,50 (Gew.-%) |
| Ethanol | 5,00 |
| Propylencarbonat | 25,00 |
| Natriumhydroxid, 32%-ig | 0,20 |
| Quaternäres Dimethylaminoethylmethacrylat-Homopolymerisat (Polyquaternium-37;50%-ig in einem Propylenglykoldicaprat-dicaprylat PPG-1-Trideceth-6 Gemisch; Salcare$^{R}$SC96) | 3,50 |
| Acid Orange 7 (C.I.-No. 15,510) | 0,15 |
| Acid Yellow 3 (C.I.-No. 47,005) | 0,10 |
| Acid Violet 43 (C.I.-No. 60,730) | 0,25 |
| Coenzym Q10 | 0,10 |
| Milchsäure, 90%-ig | ad pH 1,8 |
| Wasser | ad 100,00 |

Die Zusammensetzung wurde auf nasses Haar aufgebracht.
Nach etwa zwanzigminütiger Einwirkung wurde gewaschen, gespült und getrocknet. Es wurde ein glänzend mittelbraun gefärbtes Haar, das leicht kämmbar war, einen vollen Griff und Spannkraft aufwies, erhalten. Die Färbung blieb über vier Haarwäschen haltbar.
Eine in analoger Weise durchgeführte Färbung mit einem identischen Produkt, allerdings ohne Hinzufügung des Coenzym Q10, führte zu einer wesentlich weniger intensiven und stabilen Färbung.

**Patentansprüche**

1. Färbemittel auf Basis eines direktziehenden Haarfarbstoffs, enthaltend mindestens 0,001 bis 10 Gew.-%, bezogen auf die Zusammensetzung, mindestens eines Ubichinons der Formel (I)

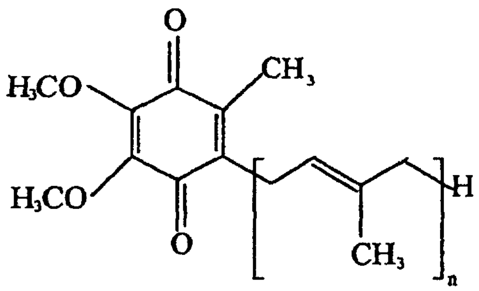

(I)

,

worin n eine Zahl von 1 bis 10 bedeutet.

2. Mittel nach Anspruch 1, worin n 10 bedeutet.

3. Mittel nach Anspruch 1, worin n 6 bedeutet.

4. Mittel nach einem oder mehreren der Ansprüche 1 bis 3, enthaltend 0,1 bis 2,5 Gew.-% mindestens eines Ubichinons.

5. Verwendung eines Ubichinons der Formel (I)

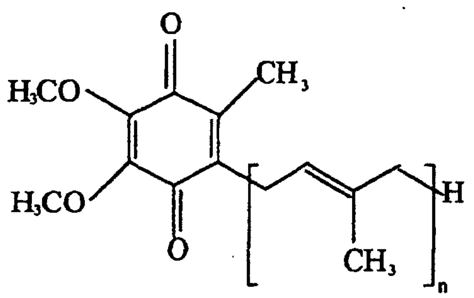

(I)

,

worin n eine Zahl von 1 bis 10 bedeutet,
in Färbemitteln auf Basis von direktziehenden Haarfarbstoffen.

## Claims

1. Coloring composition on the basis of a direct-acting hair dyestuff, comprising at least 0.001 % to 10 % by weight, calculated to the total composition, of at least one ubiquinone of the formula (I)

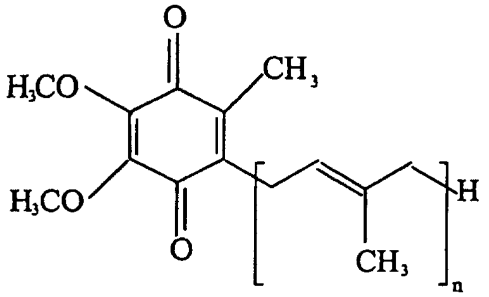

(I)

,

wherein n is a number from 1 to 10.

2. Composition according to claim 1, wherein n is 10.

3. Composition according to claim 1, wherein n is 6.

4. Composition according to one or more of claims 1 to 3, comprising 0.1 % to 2.5 % by weight, calculated to the total composition, of a ubiquinone.

5. Use of a ubiquinone of the formula (I)

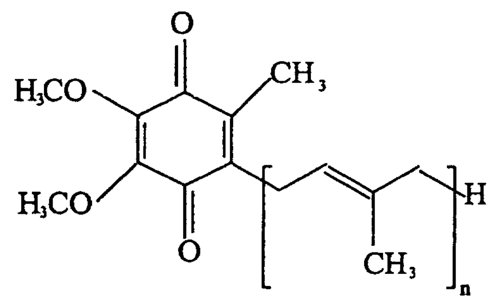

(I)

,

wherein is a number from 1 to 10,
in coloring compositions on the basis of direct-acting hair dyestuffs.

**Revendications**

1. Agent de coloration à base d'un colorant capillaire direct, qui contient au moins de 0,001 à 10 % en poids par rapport à la composition, d'au moins une ubiquinone de formule (I)

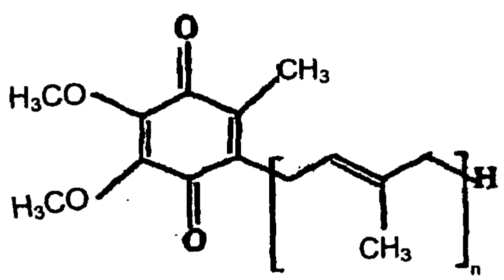

(I)

,

dans laquelle n représente un nombre de 1 à 10.

2. Agent selon la revendication 1, dans lequel n représente 10.

3. Agent selon la revendication 1, dans lequel n représente 6.

4. Agent selon l'une ou plusieurs des revendications 1 à 3, qui contient de 0,1 à 2,5 % en poids d'au moins une ubiquinone.

5. Utilisation d'une ubiquinone de formule (I)

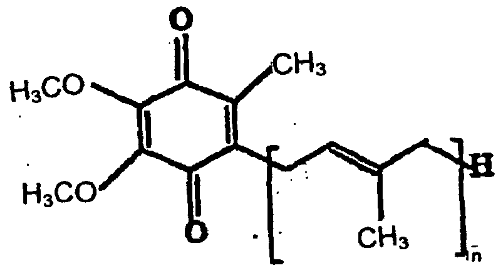

(I)

dans laquelle ne représente un nombre de 1 à 10
dans des agents de coloration à base de colorants capillaires directs.